Europäisches Patentamt

European Patent Office

Office européen des brevets

(19)

(11) Numéro de publication: **0 189 704**
**B1**

(12) **FASCICULE DE BREVET EUROPEEN**

(45) Date de publication du fascicule du brevet:
31.01.90

(21) Numéro de dépôt: 85402587.1

(22) Date de dépôt: 20.12.85

(51) Int. Cl.⁴: **C 07 H 15/04**

(54) **Procédé de préparation de maltitol cristallisé.**

(30) Priorité: 20.12.84 FR 8419600

(43) Date de publication de la demande:
06.08.86 Bulletin 86/32

(45) Mention de la délivrance du brevet:
31.01.90 Bulletin 90/5

(84) Etats contractants désignés:
BE CH DE FR GB IT LI NL SE

(56) Documents cités:
EP-A- 0 072 080
FR-A- 2 000 580
FR-A- 2 454 830
FR-A- 2 499 576

PATENTS ABSTRACTS OF JAPAN, vol. 9, no. 12 (C-261)
[1735], 18 janvier 1985, page 96 C 261; & JP - A - 59 162
953 (SHOWA DENKO K.K.) 13-09-1984
CHEMICAL ABSTRACTS, vol. 86, 1977, page 431, no.
70198v, Columbus, Ohio, US; I. KEIJI et al.:
"Determination of maltitol by thin-layer
chromatography" & KOSHIEN DAIGAKU KIYO 1976, 5,
28-31
CHEMICAL ABSTRACTS, vol. 98, no. 22, mai 1983, page
105, no. 181481z, Columbus, Ohio, US; & JP - A - 57 209
000 (JAPAN ORGANO CO., LTD.) 22-12-1982

(73) Titulaire: **Roquette Frères, F-62136 Lestrem (FR)**

(72) Inventeur: **Devos, Francis, 70 route de Merville La Motte
au Bois, Morbecque F-59190 Hazebrouck (FR)**
Inventeur: **Gouy, Pierre-Antoine, 59 Chemin Vert,
F-59130 Lambersart (FR)**

(74) Mandataire: **Koch, Gustave et al, Cabinet
PLASSERAUD 84, rue d'Amsterdam, F-75009 Paris (FR)**

## Description

L'invention a pour objet un procédé de préparation de maltitol cristallisé.

Le maltitol ou α-D-glucopyranosyl 4-D-sorbitol est le résultat de l'hydrogénation du maltose.

Il est connu de préparer du maltitol cristallisé anhydre en induisant la cristallisation dudit maltitol dans un sirop suffisamment riche en ce produit et suffisamment purifié (brevet France 2 499 576).

Un tel sirop est habituellement obtenu par hydrogénation d'un sirop riche en maltose ou par hydrogénation de maltose cristallisé. Il importe, dans ce procédé, que la matière soumise à l'hydrogénation contienne le maltose en proportion très élevée de façon à n'obtenir, après ladite hydrogénation, que très peu d'impuretés du type polyalcools à caractère glucidique, lesquelles gênent voire empêchent la cristallisation du maltitol et rendent à tout le moins délicate l'extraction de ce maltitol des sirops qui le contiennent.

On connaît de nombreux procédés pur la fabrication de sirops riches en maltose, à savoir notamment:

— celui décrit par HODGE et Coll. dans «Cereal Chemistry» n° 25, pages 19—30, janvier 1948, et qui comprend une étape de précipitation des dextrines limites par des solutions alcooliques.

— celui décrit par WOLFROM et THOMPSON dans «Methods in carbohydrate chemistry», 1962, pages 334—335 et qui comprend une étape de cristallisation répétée de l'octaacétate de maltose suivie de la cristallisation du maltose,

— celui décrit dans le brevet US 4 294 623 de MEIJI SEIKA accordé le 13.10.1981 et qui comprend une étape d'adsorption sur charbon des dextrines,

— celui décrit dans le brevet FR 2 510 581 de HAYASHIBARA déposé le 03.08.1982 et qui comprend une étape de chromatographie sur zéolithes ou résines cationiques ou anioniques,

— celui décrit dans le brevet US 4 429 122 de U.O.P. et qui comprend une étape d'ultrafiltration des sirops de maltose et, surtout,

— celui qui est décrit dans le brevet FR 2 012 831 de HAYASHIBARA déposé le 27.03.1970 et qui comprend l'utilisation combinée de plusieurs enzymes différentes, à savoir une α-amylase, une β-amylase et une isoamylase et/ou pullulanase.

Le procédé décrit dans le dernier des susdits documents est celui retenu dans le cadre du susdit brevet FR 2 499 576 pour parvenir au sirop suffisamment riche en maltose subséquemment hydrogéné puis soumis à la cristallisation.

Bien que représentant un certain progrès par rapport aux procédés décrits dans les autres documents cités, le procédé objet du brevet FR 2 499 576 — qui consiste, dans une première étape, à préparer un sirop riche en maltose mais à faible teneur en matières sèches puis, dans une deuxième étape, à élever la teneur en matières sèches de ce sirop —, présente encore plusieurs inconvénients dont notamment:

— celui d'être peu performant en raison de la faible teneur en matières sèches du produit de départ, voisine de 80 g/l, nécessaire pour obtenir une efficacité d'hydrolyse la plus élevée possible des enzymes au moment de la saccharification et impliquant une concentration importante du sirop de maltose obtenu; le susdit caractère peu performant est accentué par les pertes en maltitol inévitables dans les eaux de cristallisation qui en contrennent des quantités non négligeables,

— celui d'impliquer une rétrogradation intempestive de l'amylose, gênante pour les opérations de saccharification et de purification et due au fait que la liquéfaction doit être effectuée à des valeurs de dextrose-équivalent ou DE très faibles.

L'invention a donc pour but, surtout, de remédier à des inconvénients et de fournir un procédé du genre en question répondant mieux que ceux qui existent déjà aux divers desiderata de la pratique.

Or, il se trouve que la Société Demanderesse a réussi à mettre au point un procédé industriel nouveau fournissant, de façon aisée et avec un excellent rendement, du maltitol cristallisé d'une richesse en maltitol supérieure à 96% et, de préférence, supérieure à 97%.

Le procédé conforme à l'invention comprend successivement:

— une étape d'hydrogénation catalytique effectuée de manière en elle-même connue, d'un sirop de maltose contenant au moins 50%, de préférence de 60 à 80% en poids sur matières sèches, de maltose obtenu par saccharification enzymatique, à une matière sèche comprise entre 25 et 45%, d'un lait d'amidon liquéfié par voie acide ou enzymatique,

— une étape de fractionnement chromatographique effectuée de manière en elle-même connue du sirop de maltitol obtenu à l'étape précédente, les paramètres de cette étape de fractionnement chromatographique dont notamment

— le débit d'élution,
— le débit d'alimentation en sirop hydrogéné,
— le débit d'extraction de la fraction riche en maltitol,
— la composition des zones de désorption, d'adsorption et d'enrichissement, étant chosis de manière telle qu'on obtienne une fraction (A) riche en maltitol ayant la composition suivante, les pourcentages étant exprimés en poids sur matières sèches:

— au moins 87%, de préférence de 87 à 97,5% et, plus préférentiellement, de 87 à 95,5% de maltitol,

— une proportion de polyols de degré de polymérisation ou DP ≥ 4 inférieure à 1%, de préférence inférieure à 0,7% et, plus préférentiellement encore, inférieure à 0,6%,

le complément à 100% étant constitué par du sorbitol et du maltotriitol,

— une étape de concentration de la fraction (A) riche en maltitol à une teneur en matières sèches propre à permettre la formation des cristaux de maltitol, comprise de préférence entre 75 et 92% de matières sèches,

— une étape de cristallisation et de séparation des cristaux de maltitol et

– une étape de recyclage des eaux-mères de cristallisation en tête de l'étape de fractionnement chromatographique, ce recyclage des eaux-mères de cristallisation permettant une extraction presque quantitative du maltitol formé lors de l'étape d'hydrogénation du sirop de maltose.

Les paramètres de l'étape de saccharification comprennent le type et la quantité d'enzymes employées, la température, la durée d'action et autres.

L'efficacité du fractionnement chromatographique permet d'exclure de la fraction maltitol les produits hydrogénés de DP (degré de polymérisation) supérieur ou égal à 4 d'une façon quasi totale, même si les sirops soumis au fractionnement en contiennent des quantités notables, par exemple comprises entre 5 et 40%. On peut donc avoir recours à des hydrolysats d'amidon dont la richesse en maltose n'est que de 50%. Or, pour préparer de tels sirops, on peut mette en œuvre des laits d'amidon à des teneurs en matières sèches élevées, en tout cas comprises entre 25 et 45% et voisines de 40%, comme celles qu'il est habituel de mettre en œuvre dans les glucoseries-dextroseries.

Grâce au procédé conforme à l'invention,

– les volumes à traiter sont bien moindres que dans les procédés antérieurs,

– l'énergie nécessaire à l'évaporation de l'eau se trouve fortement réduite,

– la liquéfaction de l'amidon peut se faire à un DE (Dextrose-Equivalent) supérieur à 2, compatible avec une absence de rétrogradation de l'amidon,

– l'emploi d'enzymes comme l'isoamylase ou la pullulanase peut être évité,

– les hautes pressions osmotiques occasionnées par la concentration élevée des sirops mis en œuvre mettent ceux-ci à l'abri de toute contamination microbienne.

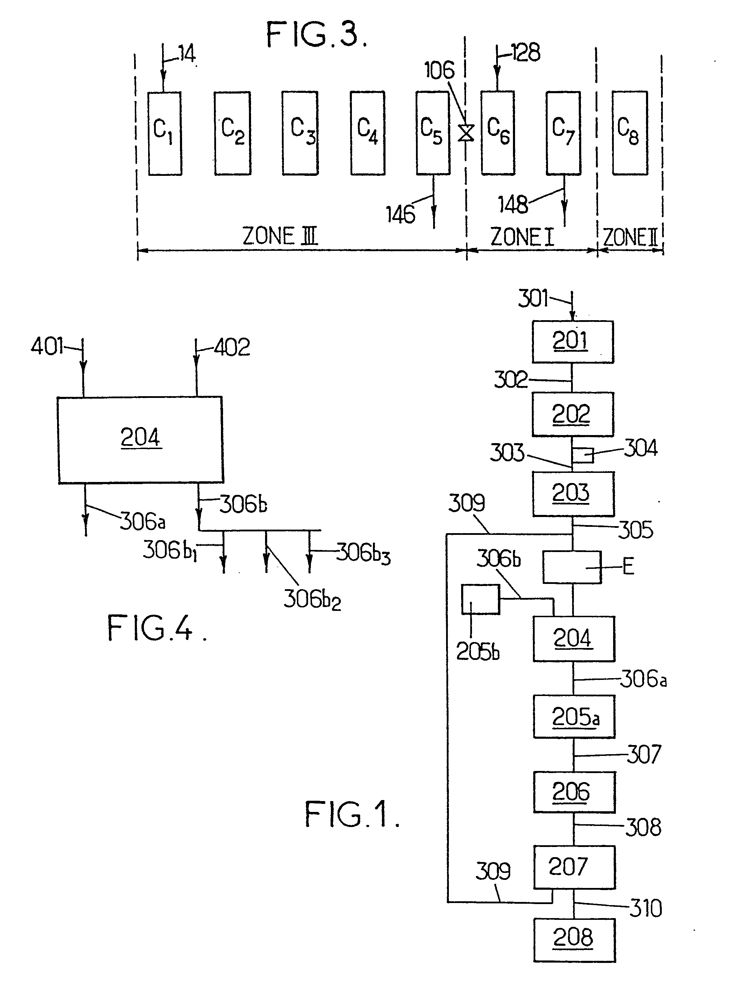

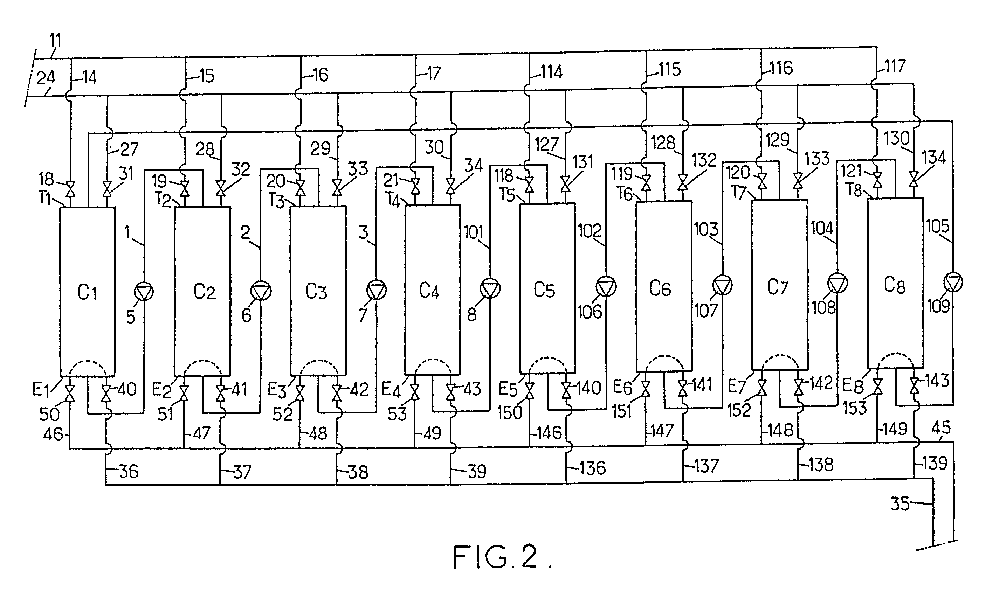

Le procédé conforme à l'invention peut être mis en œuvre à l'aide de l'installation schématiquement représentée à la figure 1 et qui comprend:

– une enceinte 201 à l'intérieur de laquelle on procède à la liquéfaction de l'amidon,

– une enceinte 202 à l'intérieur de laquelle on procède à la saccharification de l'amidon,

– une enceinte 203 à l'intérieur de laquelle on procède à l'hydrogénation catalytique,

– un dispositif de concentration E,

– une enceinte 204 de séparation chromatographique

– une ou plusieurs enceintes 205a, 205b..., permettant de concentrer aux teneurs en matières sèches souhaitées, en alternance ou chacune en continu, les diverses fractions issues de la chromatographie et notamment la fraction riche en maltitol étant recueillie dans l'enceinte 205a,

– une enceinte 206 à l'intérieur de laquelle s'effectue la cristallisation du maltitol qui a pu commencer dans l'enceinte 205a,

– une enceinte 207 permettant d'effectuer la séparation des cristaux formés d'avec leurs eaux-mères,

– une enceinte 208 permettant d'effectuer le séchage des cristaux extraits de l'enceinte 207.

L'enceinte 201 est alimentée par une canalisation 301 en lait d'amidon ou de fécule additionné d'acide dans le cas d'une liquéfaction dite acide, ou d'une α-amylase dans le cas d'une liquéfaction enzymatique, dans les conditions de température, de pH, de taux d'enzyme et de calcium, connues de l'homme de l'art pour obtenir un DE (dextrose équivalent) égal ou supérieur à 2.

L'enceinte 202 est alimentée par une canalisation 302 en sirop d'amidon liquéfié sortant de l'enceinte 201. Avant son entrée dans l'enceinte 202, on ajoute au sirop sortant de l'enceinte 201, une β-amylase de malt et on choisit les paramètres de la saccharification de telle manière qu'on obtienne une richesse en maltose d'au moins 50% et, de préférence, d'au moins 60% à la sortie de l'enceinte 202. Les paramètres de la saccharification enzymatique sont notamment la quantité d'enzyme employée, la température, le pH et la durée de l'amylolyse.

L'enceinte 203 est alimentée à partir de l'enceinte 202 par une canalisation 303 en sirop saccharifié puis filtré et déminéralisé dans un dispositif 304 placé sur la canalisation 303. On procède dans l'enceinte 203 à l'hydrogénation catalytique du sirop de maltose dans les conditions bien connues de l'homme de l'art, notamment avec des catalyseurs au ruthénium ou au nickel de Raney.

De préférence, l'étape d'hydrogénation est effectuée avec un catalyseur au nickel de Raney, à une pression d'hydrogène supérieure à $20 \, kg/cm^2$ et comprise de préférence entre 40 et $70 \, kg/cm^2$ et à une température d'environ 100 à 150°C. L'hydrogénation est poursuivie jusqu'à ce que la teneur en sucres réducteurs du sirop hydrogéné soit inférieure à 2%, de préférence inférieure à 1% et, plus préférentiellement encore, inférieure à 0,5% (la teneur en sucres réducteurs étant définie en poids d'équivalent dextrose par rapport à la matière sèche).

L'enceinte 204 est alimentée à partir du dispositif de concentration ou évaporateur E recevant par une canalisation 305 le sirop hydrogéné purifié sortant de l'enceinte 203. La canalisation 305 reçoit également une canalisation 309 issue de l'enceinte 207 ci-dessous décrite.

Comme indiqué plus haut, on procède dans l'enceinte 204 au fractionnement chromatographique du sirop de maltitol issu de l'enceinte 203.

On achemine respectivement vers les enceintes 205a, 205b et par les canalisations 306a, 306b..., les fractions sortant de l'enceinte 204.

La fraction très riche en maltitol est acheminée vers l'enceinte 205a.

L'étape de fractionnement chromatographique peut être effectuée de manière connue en soi, de façon discontinue ou continue (lit mobile simulé), sur des adsorbants du type résines cationiques fortement acides, chargées en ions alcalins ou alcalino-terreux ou encore du type zéolithes chargées en ions ammonium, sodium, potassium, calcium, baryum, strontium, etc.

Des exemples de tels procédés de séparation chromatographique sont donnés dans les brevets US 3 044 904, US 3 416 961, US 3 692 582, FR 2 391 754, FR 2 099 336, US 2 985 589, US 4 024 331, US 4 226 977, US 4 293 346, US 4 157 267, US 4 182 633, US 4 332 633, US 4 405 445, US 4 412 866 et US 4 422 881.

Suivant un mode de réalisation préféré, l'étape de séparation est effectuée par mise en œuvre du procédé et de l'appareillage décrite dans le brevet US 4 422 811 et son correspondant français N° 7 910 563 dont la Société Demanderesse est titulaire.

Quel que soit le procédé de séparation chromatographique retenu, on a recours, de préférence, à titre d'adsorbant, à une résine cationique forte mise sous forme calcium et ayant un taux de divinylbenzène d'environ 4 à 10%.

Le choix des paramètres de l'étape de chromatographie, dont notamment:
— le débit d'élution,
— le débit d'alimentation en sirop hydrogéné,
— le débit d'extraction de la fraction riche en maltitol,
— la composition des zones de désorption, d'adsorption et d'enrichissement,
se trouve expliqué et illustré dans l'exemple.

Le procédé ainsi décrit permet d'obtenir des sirops de maltitol (A) présentant une richesse au moins égale à 87% de maltitol et contenant moins de 1% de produits de degré de polymérisation supérieur ou égal à 4. Ils sont, de façon plus précise, les pourcentages étant exprimés en poids sur matières sèches, composés:
— d'au moins 87%, de préférence de 87 à 97,5% et, plus préférentiellement encore, de 87 à 95,5% de maltitol,
— d'une proportion inférieure à 1%, de préférence inférieure à 0,7% et, plus préférentiellement encore, inférieure à 0,6% de polyols de degré de polymérisation supérieur ou égal à 4, le complément à 100% étant constitué par du sorbitol et du maltotriitol,
— de préférence d'une proportion en sorbitol inférieure à 5% et, plus préférentiellement encore, inférieure à 2%,
— d'une teneur en maltotriitol généralement comprise entre 2,5 et 13% en poids.

Ce procédé conduit par ailleurs à la production concomitante d'un sirop enrichi en maltotriitol et à celle d'un sirop composé de produits hydrogénés à haut poids moléculaire.

Ces deux types de sirop sont extraits de l'enceinte de chromatographie par la canalisation 306 b.

Comme indiqué plus haut, l'enceinte 205 a est alimentée en sirop très riche en maltitol (A) issu de l'enceinte de chromatographie par la canalisation 306 a. Cette enceinte 205 a est agencée de manière à permettre de concentrer le sirop riche en maltitol. De préférence, cette concentration est poursuivie jusqu'à une matière sèche comprise entre 75 et 92%. Cette concentration peut être effectuée de façon continue ou discontinue et, de préférence, sous pression réduite. Lors de cette étape, on peut amorcer ou non la formation des cristaux.

L'enceinte 206 est agencée de manière à permettre d'achever la cristallisation amorcée dans l'enceinte 205 a ou à permettre é cette cristallisation de s'effectuer complètement.

Elle est habituellement munie des dispositifs permettant de mener à bien cette opération, à savoir des moyens d'agitation et de refroidissement de la masse cristalline, lesquels moyens peuvent être mis en œuvre de diverses manières mais de toute façon de manière telle que les masses cristallines soient refroidies de façon contrôlée et homogène.

Elle est reliée à l'enceinte 205 a par une canalisation 307, une canalisation 308 permettant d'acheminer les masses cristallisées vers l'enceinte 207.

L'enceinte 207 comprend généralement un dispositif à action centrifuge apte à séparer des cristaux de leurs eaux-mères et muni d'un dispositif de lavage desdits cristaux de façon à les porter à une pureté chimique suffisante.

L'évacuation des eaux-mères s'effectue par une canalisation 309 qui ramène lesdites eaux-mères à la canalisation 305, immédiatement en aval de l'enceinte d'hydrogénation 203.

C'est par une canalisation 310 que les cristaux isolés dans l'enceinte 207 sont acheminés vers l'enceinte 208.

L'enceinte 208 comporte des moyens de séchage permettant d'éliminer l'humidité résiduelle des cristaux; il peut s'agir de dispositifs statiques ou pneumatiques fonctionnant sous pression positive ou négative; elle est préférentiellement du type à veine d'air chaud transportant ou maintenant en suspension lesdits cristaux.

Ces cristaux sont obtenus facilement sous une forme pratiquement anhydre (teneur en eau inférieure à 0,5%); ils constituent une poudre non hygroscopique, s'écoulant tout à fait librement.

Les eaux-mères de la cristallisation du maltitol sont réincorporées préférentiellement avant l'étape de concentration dans le flot de sirop issu de l'enceinte d'hydrogénation 203. Le mélange résultant est porté dans le dispositif E à une concentration permettant un fonctionnement normal et économique de l'installation de fractionnement chromatographique.

Le procédé conforme à l'invention permet, grâce au recyclage des eaux-mères, d'extraire avec une efficacité encore jamais atteinte et sous sa forme cristallisée, la quasi totalité du maltitol présent dans un sirop de maltitol.

L'invention pourra encore être mieux comprise à l'aide de l'exemple qui est relatif à un mode de réalisation avantageux de l'invention sans en limiter la portée.

Exemple

On a recours à une installation telle que représentée à la figure 1.

Un lait d'amidon de forment d'une teneur en matières sèches de 37%, est liquéfié dans l'enceinte 201 à un pH de 6,3, à une température de 108°C et avec une addition de 0,3‰ d'enzyme

liquéfiante du type de celle commercialisée par la Société NOVO sous la marque «THERMAMYL». A la sortie de l'installation de liquéfaction, on a procédé à un choc thermique de 10 secondes à 130°C. Le DE en sortie de liquéfaction était égal à 5,0.

On ajuste le pH à la sortie de l'installation de liquéfaction à une valeur de 5,5 et on ajoute 0,55‰ de β-amylase de malt commercialisée par la Société FINNSUGAR sous la dénomination «SPEZYME BBA 1500».

La saccharification est effectuée dans l'enceinte 202 à ce pH pendant 48 heures à 57°C.

En fin de saccharification, l'analyse par chromatographie liquide montre la présence de:

| | |
|---|---|
| Dextrose | 2,3% en poids |
| Maltose | 61,3% en poids |
| Trisaccharides | 7,5% en poids |
| Produits de DP 4 à DP 10 | 6,2% en poids |
| Produit de DP > 10 | 22,7% en poids. |

L'hydrogénation est effectuée dans l'enceinte 203 (les paramètres étant ceux décrits plus haut), suivie d'une purification et d'une concentration dans le dispositif E; on obtient un sirop riche en maltitol dont la composition est la suivante:

| | |
|---|---|
| Sorbitol | 3,3% en poids |
| Maltitol | 60,4% en poids |
| Trisaccharides | 9,2% en poids |
| Produits de DP 4 à DP 10 | 7,0% en poids |
| Produit de DP > 10 | 20,1% en poids. |

On procède au fractionnement de cet hydrolysat riche en maltose hydrogéné dans l'installation 204 de séparation chromatographique continue dont les détails de la construction et du fonctionnement sont ceux décrits dans le brevet US 4 422 881 et dans le brevet correspondant français N° 7 910 563, ces détails n'étant repris ici que dans la mesure où la compréhension du texte l'exige.

Cette installation 204 comprend, comme montré à la figure 2 du brevet américain (reprise ici en tant que figure 2, pour l'explicitation détaillée de laquelle on se reportera au brevet américain), huit colonnes ou étages $C_1$ à $C_8$ de 200 litres chacune, garnies d'adsorbant du type résines cationiques fortes sous forme calcium et de fine granulométrie (0,2 à 0,4 millimètres).

De par le calage des électrovannes, on établit dans cette installation une zone I de désorption de deux étages, une zone II d'adsorption d'un étage et une zone III d'enrichissement et de séparation des dextrines limites hydrogénées et du maltotriitol, de cinq étages comme montré figure 3 qui est une représentation schématique de l'installation selon la figure 2 et sur laquelle on n'a fait figurer que:

— les colonnes $C_1$ à $C_8$,
— le dispositif de fermeture, en l'occurrence l'électrovanne 106,
— les canalisations d'alimentation en sirop de maltitol à fractionner et en eau, montrées respectivement en 14 (correspondant à la canalisation 305, figure 1) et 128 et
— la canalisation 148 d'extraction de sirop enrichi en maltitol d'une part, et la canalisation 146 d'extractions successivement de sorbitol, de dextrines limites et de maltotriitol, d'autre part.

Le dispositif de fermeture 106 (notamment une électrovanne) maintient dans la configuration adoptée, une étanchéité totale entre, d'une part, la zone III, qui est une zone d'enrichissement à l'extrémité de laquelle sont donc récupérés successivement le reliquat de sorbitol fortement adsorbé, les dextrines limites hydrogénées, puis la fraction enrichie en maltotriitol et, d'autre part, la zone I de désorption du maltitol, zone en tête de laquelle est introduite l'eau de désorption.

Ce dispositif de fermeture assure le sens du passage de la phase liquide sur l'adsorbant sélectif et évite surtout la contamination du maltitol enrichi par des traces de dextrines limites hydrogénées à haut poids moléculaire, dont la vitesse de migration au sein de la résine est largement supérieure à celle du maltitol.

Une minuterie ajustée à 23'30'' assure pour les débits indiqués ci-après une alimentation en eau suffisante pour effectuer la désorption de la totalité du maltitol au premier étage ou première colonne de la zone I de désorption, et une alimentation en un volume d'hydrolysat d'amidon hydrogéné riche en maltitol compatible avec le volume d'adsorbant et sa capacité d'adsorption, de façon à obtenir un taux d'extraction de maltitol au moins égal à 90% de maltitol présent dans l'hydrolysat hydrogéné et cela à une richesse au moins égale à 87% en maltitol vrai. Le sirop obtenu a une teneur inférieure à 0,5% de produits de DP supérieur ou égal à 4.

Les susdits taux d'extraction et puretés sont maintenus constants en ajustant le débit de la pompe d'extraction non montrée du maltitol adsorbé. La sortie des fractions «dextrines limites hydrogénées» et «maltotriitol enrichi» s'effectue à pression atmosphérique et son débit constant résulte de la différence entre les débits d'alimentation et le débit d'extraction.

L'hydrolysat d'amidon hydrogéné riche en maltitol qui est introduit dans l'installation en tête de la zone d'enrichissement III, présente, comme indiqué plus haut, une teneur en matières sèches de 51,5%. La température à l'intérieur des colonnes de séparation est maintenue à environ 90°C.

La figure 4 montre schématiquement en 204 l'installation des figures 2 et 3, les mêmes références désignant les mêmes éléments pour les parties communes que dans la figure 1. L'installation de chromatographie 204 comporte une canalisation 306b par laquelle sont éliminés les excédents d'eau contenant une fraction importante de sorbitol et la fraction dextrines limites hydrogénées à poids moléculaire supérieur ou égal à DP 4; ces extraits sont à faible teneur en matières sèches et sortent par les canalisations $306b_1$ et $306b_2$.

L'alimentation en eau s'effectue par une canalisation 402.

Les flèches portées sur les canalisations indiquent le sens de la circulation.

L'ensemble de chromatographie 204 fonctionne comme suit:
— l'hydrolysat d'amidon hydrogéné devant être soumis au fractionnement chromatographique est acheminé par la canalisation 401 à un débit de 90 litres/heure et présente une teneur en matières sèches de 51,5%.

– l'eau est amenée par la canalisation 402, avec un débit de 430 litres/heure.

– le maltitol enrichi exempt de dextrines limites hydrogénées est récupéré par la canalisation 306a avec un débit de 145 litres/heure, sa teneur moyenne en matières sèches étant de 23%,

– la quantité totale de liquides extraits par ailleurs de l'installation l'est avec un débit total de 375 litres/heure, se composant successivement:

* d'une fraction d'excédent d'eau, extraite par la canalisation $306b_1$, contenant, à faible concentration et haute pureté, du sorbitol et d'une fraction à faible concentration et à richesse élevée en dextrines limites hydrogénées à DP supérieur ou égal à 4 extraite par la canalisation $306b_2$, l'ensemble représentant un équivalent de 305 litres/heure, la teneur en matières sèches étant de 4,5%; ces fractions correspondent aux 18,5 premières minutes du cycle,

* d'une fraction de maltotriitol enrichie, d'un équivalent de 70 litres/heure acheminée par une canalisation $306b_3$ vers une installation de purification non montrée, la teneur en matières sèches de cette fraction étant de 8,2%; cette fraction correspond aux cinq dernières minutes du cycle.

Les tableaux I et II ci-dessous résument les conditions caractérisant le fonctionnement du dispositif de fractionnement chromatographique.

Tableau I

|  | Sirop maltitol | Eau | Total |
|---|---|---|---|
| Débit | 90 l/h | 430 l/h | 520 l/h |
| Densité | 1,25 kg/l |  |  |
| Matières sèches | 51,5% |  |  |
| Débit massique | 56,3 kg/h |  | 56,9 kg/h |
| Richesse en maltitol | 60,4% |  |  |
| Débit massique en maltitol | 34 kg/h |  | 34 kg/h |

Les effluents extraits de l'installation sont identifiés dans le tableau II.

Tableau II

|  | Maltitol enrichi | Sorbitol puis dextrines limites hydrogénées | Maltotriitol | Total |
|---|---|---|---|---|
| Débit | 145 l/h | 305 l/h | 70 l/h | 520 l/h |
| Densité | 1,11 kg/l | 1,02 kg/l | 1,03 kg/l |  |
| Matières sèches | 23% | 4,5% | 8,2% |  |
| Débit massique | 37 kg/h | 14,0 kg/h | 5,9 kg/h | 56,9 kg/h |
| Richesse en maltitol | 90,5% | 2% | 3,9% |  |
| Débit massique en maltitol | 33,5 kg/h | 0,28 kg/h | 0,23 kg/h | 34,1 kg/h |

Ce résultat correspond à un taux d'extraction massique de:

$$\frac{37}{56,3} = 67\% \text{ de sirop de maltitol enrichi}$$

à 90,5% et de

$$\frac{33,5}{34} = 98,5\% \text{ d'extraction du maltitol}$$

L'analyse de la fraction maltitol enrichi donne les résultats suivants:

– Sorbitol          1,3% en poids
– Maltitol          90,5% en poids
– DP 3          7,8% en poids
– DP ≥ 4          0,4% en poids.

L'analyse de la fraction sirop de maltotriitol enrichi donne les résultats suivants:

– Sorbitol          0,4% en poids
– Maltitol          3,9% en poids
– Maltotriitol          51 % en poids
– Maltotétraitol          10,6% en poids
– Produits de DP égal à 5    9,5% en poids
– Produits de DP > 5    24,6% en poids.

On peut constater, à partir de ces différentes mesures qu'il a été possible, par ce procédé de fractionnement à partir d'un sirop standard obtenu par saccharification à la β-amylase seule et aux concentrations coutumières élevées, d'extraire à 98,5% le maltitol à une richesse de 90,5%.

Le sirop de maltitol obtenu est caractérisé par l'absence quasi totale de polymères hydrogénés du glucose de DP ≥ 4.

On a pu extraire conjointement un sirop enrichi en maltotriitol à une richesse de 51%.

On a concentré dans l'enceinte 205a, sous pression réduite jusqu'à une teneur de 90% de matières sèches à une température de 80°C, la fraction riche en maltitol. Ce sirop a été recueilli dans l'enceinte de cristallisation qui est munie d'une double enveloppe. Après quatre heures de maintien à la température de 75°C, on voit apparaître un début de cristallisation très régulier (nucléation spontanée).

On a procédé ensuit au refroidissement de l'enceinte de cristallisation à une vitesse de 1°C/heure de 75°C à 25°C en 50 heures.

Les masses cristallines obtenues sont essorées dans les conditions suivantes:

– poids de masse cristalline à 90% de matières sèches:          102,2 kg

– quantité d'eau de clairçage:          45 litres

– durée totale d'un cycle (y compris les durées d'accélération, de freinage et de débatissage du gâteau): 30 minutes.

Les résultats moyens obtenus s'établissaient comme suit (moyenne pour 10 cycles d'essorage):
- poids moyen des cristaux humides: 63,2 kg
- humidité des cristaux: 5,4%
- richesse des cristaux en maltitol: 99%
- richesse des cristaux en sorbitol: 0,5%
- richesse des eaux-mères en maltitol: 75%
- richesse des eaux-mères en sorbitol: 5,6%
- richesse des eaux-mères en maltotriitol: 19,5%
- richesse des eaux-mères en produits de DP $\geq$ 4: 0,9%
- matière sèche moyenne des eaux-mères: 38,4%
- masse moyenne d'eaux-mères: 83,8 kg
- rendement moyen de cristallisation: 65%
(maltitol cristallisé anhydre par rapport à matière sèche totale soumise à la cristallisation).

C'est après 48 heures de fonctionnement du dispositif de fractionnement chromatographique dans les conditions précitées que débute la phase de recyclage des eauxmères de cristallisation prévue conformément à l'invention.

Les eaux-mères de cristallisation accumulées pendant 48 heures sont recyclées par la canalisation 309 immédiatement en amont de l'enceinte E d'évaporation du sirop de maltitol alimentant le dispositif de chromatographie 204.

Le recyclage a été effectué à raison de 100 kg de matières sèches d'eaux-mères pour 330 kg de matières sèches de sirop de maltitol à 60,4% de richesse issu de l'étape d'hydrogénation, soit 23% environ de la matière sèche du sirop alimentant le dispositif de chromatographie.

Ce pourcentage correspond à un état d'équilibre du procédé.

Dans ces conditions, la totalité des eaux-mères obtenues à l'étape de cristallisation subit à nouveau le fractionnement chromatographique.

La composition du sirop alimentant l'étape de chromatographie n'a été que peu modifiée, puisque s'établissant à:

| sorbitol | : | 3,8% |
| maltitol | : | 63,8% |
| maltotriitol | : | 11,4% |
| DP $\geq$ 4 | : | 21 %. |

La richesse en maltitol de l'alimentation n'a donc pas sensiblement changé, tout au plus peut-on remarquer une légère augmentation de la teneur en sorbitol et maltotriitol assortie d'une diminution corrélative des produits hydrogénés à haut poids moléculaire (DP $\geq$ 4).

Le dispositif de fractionnement chromatographique a fonctionné dans les conditions d'alimentation en eau et sirop, et dans les conditions d'extraction en sirop de maltitol et autres avec les débits mentionnés aux tableaux I et II ci-dessus.

Après 24 heures de fonctionnement, l'équilibre étant atteint, l'effluent maltitol a montré la composition suivante:

| sorbitol | : | 1,9% |
| maltitol | : | 90,5% |
| DP 3 | : | 7,4% |
| DP $\geq$ 4 | : | 0,2% |

La concentration puis la cristallisation de ce sirop dans les conditions déjà décrites a fourni à nouveau, après essorage, du maltitol cristallisé avec un rendement de 65% par rapport à la matière sèche totale soumise à la cristallisation.

Ces cristaux ont été séchés sur séchoir à lit fluidisé. Ils ont révélé les caractéristiques suivantes:

Tableau III

| H$_2$O: | 0,3% |
| pouvoir rotatoire (solution aqueuse à 10%) à 20°C raie D du sodium: | 106° |
| sucres réducteurs: | < 0,01% |
| *température de fusion au pic: | 150,6°C |
| richesse par chromatographie liquide haute pression: | 98,5% |

*caractéristique thermique relevée sur appareil D.S.C. SETARAM 111, 50 mg de substance, vitesse de chauffage de 2°C/mn.

Ils sont non hygroscopiques et forment une poudre qui s'écoule librement.

Il résulte de ce qui précède que le procédé conforme à l'invention de fabrication de maltitol cristallisé permet une extraction quasi totale du maltitol formé lors de l'étape d'hydrogénation car les eaux-mères de cristallisation peuvent être totalement recyclées et car l'unique perte de maltitol a lieu lors de l'étape de chromatographie où seule une très petite fraction de celui-ci se mélange à la partie riche en maltotriitol ainsi qu'aux autres fractions enrichies qu'il est possible de séparer. Or, à ce niveau, les rendements d'extraction du maltitol sont pratiquement quantitatifs puisque 98,5% du maltitol présent dans le sirop d'alimentation du système de fractionnement chromatographique se retrouve dans la fraction enrichie en maltitol soumise à la cristallisation.

Ainsi donc, par ce nouveau procédé, la quasi totalité du maltose formé lors d'une hydrolyse enzymatique d'amidon peut être valorisée par une extraction sous forme de maltitol cristallisé de haute pureté.

Comme il va de soi et comme il résulte d'ailleurs déjà de ce qui précède, l'invention ne se limite nullement à ceux de ses modes d'application et de réalisation qui ont été plus spécialement envisagés; elle en embrasse, au contraire, toutes les variantes.

## Revendication

Procédé de préparation de maltitol cristallisé, caractérisé par le fait qu'il comprend successivement:
- une étape d'hydrogénation catalytique effectuée de manière en elle-même connue, d'un sirop de maltose contenant au moins 50%, de préférence de 60 à 80% en poids sur matières sèches, de maltose obtenu par saccharification enzymatique, à une matière sèche comprise entre 25 et 45%, d'un lait d'amidon liquéfié par voie acide ou enzymatique,
- une étape de fractionnement chromatographique effectuée de manière en elle-même connue du sirop de maltitol obtenu à l'étape précédente, les paramètres de cette étape de fractionnement chromatographique dont notamment
– le débit d'élution,
– le débit d'alimentation en sirop hydrogéné,
– le débit d'extraction de la fraction riche en maltitol,
– la composition des zones de désorption, d'adsorption et d'enrichissement,
étant choisis de manière telle qu'on obtienne une fraction (A) riche en maltitol ayant la composition suivante, les pourcentages étant exprimés en poids sur matières sèches:
– au moins 87%, de préférence de 87 à 97,5% et, plus préférentiellement, de 87 à 95,5% de maltitol,
– une proportion de polyols de degré de polymérisation ou DP ≥ 4 inférieure à 1%, de préférence inférieure à 0,7% et, plus préférentiellement encore, inférieure à 0,6%,
le complément à 100% étant constitué par du sorbitol et du maltotriitol,
– une étape de concentration de la fraction (A) riche en maltitol à une teneur en matières sèches propre à permettre la formation des cristaux de maltitol, comprise de préférence entre 75 et 92% de matières sèches,
– une étape de cristallisation et de séparation des cristaux de maltitol et
– une étape de recyclage des eaux-mères de cristallisation en tête de l'étape de fractionnement chromatographique, ce recyclage des eaux-mères de cristallisation permettant une extraction presque quantitative du maltitol formé lors de l'étape d'hydrogénation du sirop de maltose.

**Patentanspruch**

1. Verfahren zur Herstellung von kristallisiertem Maltit, dadurch gekennzeichnet, daß man nacheinander durchführt:
– eine Stufe der in an sich bekannter Weise durchgeführten katalytischen Hydrierung eines Maltosesirups, enthaltend mindestens 50%, vorzugsweise 60 bis 80 Gew.-% Maltose, bezogen auf Trockensubstanz, erhalten durch enzymatische Verzuckerung einer Stärkemilch mit einer Trockensubstanz zwischen 25 und 45%, die auf saurem oder enzymatischem Wege verflüssigt wurde,
– eine Stufe der in an sich bekannter Weise durchgeführten chromatographischen Fraktionierung des in der vorhergehenden Stufe erhaltenen Maltitsirups, wobei die Parameter dieser chromatographischen Fraktionierungsstufe, wovon insbesondere zu nennen sind
– der Elutionsdurchsatz
– der Zufuhrdurchsatz an hydriertem Sirup,
– der Extraktionsdurchsatz der Maltit-reichen Fraktion,
– die Zusammensetzung der Desorptions-, Adsorptions- und Anreicherungszonen,
derart ausgewählt sind, daß man eine an Maltitreiche Fraktion (A) der folgenden Zusammensetzung erhält, wobei die Prozentsätze als Gewichtsprozent, bezogen auf Trockensubstanz, angegeben sind:

– mindestens 87%, vorzugsweise 87 bis 97,5% und ganz bevorzugt 87 bis 95,5%, Maltit,
– einen Anteil an Polyolen mit einem Polymerisationsgrad oder DP > 4 unterhalb von 1%, vorzugsweise unterhalb von 0,7% und besonders bevorzugt unterhalb von 0,6%,
wobei die Ergänzung auf 100% aus Sorbit und Maltotriitol besteht,
– eine Konzentrationsstufe der an Maltit-reichen Fraktion (A) auf einen Gehalt an Trockensubstanz, der geeignet ist, die Bildung von Maltitkristallen zu ermöglichen, vorzugsweise zwischen 75 und 92% Trockensubstanz,
– eine Kristallisations- und Abtrennstufe der Maltitkristalle und
– eine Recyclisierungsstufe der Kristallisationsmutterlaugen zum Kopf der chromatographischen Fraktionierungsstufe, wobei diese Rückführung der Kristallisationsmutterlaugen eine fast quantitative Extraktion des Maltits, der während der Hydrierungsstufe des Maltosesirups gebildet wurde, erlaubt.

**Claim**

1. Process for preparing crystalline maltitol, characterized by the fact that it comprises successively:
– a catalytic hydrogenation step performed in a manner known in itself, of a maltose syrup containing at least 50%, preferably from 60 to 80% by weight on dry matter, of maltose obtained by enzymatic saccharification, with a dry matter content comprised between 25 and 45%, of a starch milk liquefied by the acid or enzymatic route,
– a chromatographic fractionation step performed in a manner known in itself, of the maltitol syrup obtained at the preceding step, the parameters of this chromatographic fractionation step among which namely
– the elution rate,
– the rate of feeding with hydrogenated syrup,
– the rate of extraction of the fraction rich in maltitol,
– the composition of the zones of desorption, adsorption and enrichment,
being selected in order to obtain a fraction (A) rich in maltitol having the following composition, the percentages being expressed by weight to dry matter:
– at least 87%, preferably from 87 to 97.5% and, more preferably, from 87 to 95.5% of maltitol,
– a proportion of polyols of degree of polymerization or DP > 4 less than 1%, preferably less than 0.7% and, still more preferably, less than 0.6%,
the complement to 100% being constituted by sorbitol and maltotriitol,
– a step of concentration of the fraction (A) rich in maltitol to a dry matter content suitable for permitting the formation of maltitol crystals, generally comprised between 75 and 92% of dry matter
– a step of crystallization and separation of the maltitol crystals and

— a step recycling the crystallization mother-liquors to the head of the chromatographic fractionation step, this recycling of the crystallization mother-liquors enabling an almost quantitative extraction of the maltitol formed during the hydrogenation step of the maltose syrup.

EP 0189704 B1

FIG.3.

ZONE III · ZONE I · ZONE II

FIG.4.

FIG.1.

11

FIG.2.